# EUROPEAN PATENT APPLICATION

(11) **EP 1 607 475 A1**
(43) Date of publication of application: **21.12.2005**
(21) Application number: 04721646.0
(22) Date of filing: 18.03.2004
(51) Int. Cl.: C12M 1/34, B65D 79/02

(54) **OXYGEN INDICATOR AND PACKAGED MATERIAL**

(30) Priority: 20.03.2003 JP 2003078768; 30.09.2003 JP 2003341181
(71) Applicant: Asahi Kasei Life & Living Corporation, Tokyo, 100-8440 (JP)
(72) Inventor: SAKURAI, Kazuaki, Suzuka-shi, Mie-ken 5130824 (JP); KAWASHIMA, Masahiko, Suzuka-shi, Mie-ken 5130846 (JP); MATSUKI, Yutaka, Kameyama-shi, Mie-ken 5190105 (JP); UEDA, Shigeru, Tagata-gun, Shizuoka 4102114 (JP); TAKAHASHI, Mamoru, Sunto-gun, Shizuoka 4110903 (JP)
(74) Representative: HOFFMANN EITLE
(86) International application number: PCT/JP2004/003643
(87) International publication number: WO 2004/083360

(57) **Abstract**

An oxygen indicator using an optical absorption spectral change reaction caused by a substrate in the presence of oxygen via an enzymatic catalysis, which comprises an oxygen sensitive solution containing at least a coloring substrate, an oxidoreductase, and a reducing agent capable of reducing the oxidized coloring substrate.

## Description

### TECHNICAL FIELD

The present invention relates to an oxygen indicator using an enzyme(s) and a package having the oxygen indicator.

### BACKGROUND ART

In addition to the conventional eating habit in which food materials are purchased at supermarkets and the like and cooked and eaten at home, people have recently become more and more inclined to have a habit in which they purchase cooked food and the like prepared in the backyards of supermarkets, etc. or in central kitchens, etc. and eat it at home, because of their intention to simply and easily do household chores, particularly cooking, because little time can be taken for cooking in dual-income households, because more time are needed for enjoying hobbies, or for other like reasons.

On the other hand, for cooked food to be sold in supermarkets and convenience stores, many kinds of products have been actively developed as meeting the preference of consumers in taste, quantity, etc. of an individual foodstuff and carted to market emphasizing the convenience of being already cooked. Furthermore, distributors of daily food in supermarkets, convenience stores and the like try to provide daily food and the like having reduced amounts of food preservatives and the like for seeking tastiness of food materials themselves that are strongly demanded by consumers and meeting requirements for security, safety and health. However, reduction in food preservatives causes the food to be rotten earlier, thus requiring measures for safety of food. Furthermore, from studies on rotting of food, it is well known that the influence of oxygen in the air is significant. Therefore, various methods have been tried for packaging a food while keeping the inside of the package under oxygen-free conditions. Methods for keeping the inside of the package under oxygen-free conditions, for the purpose of preventing the food from being rotten, include vacuum packaging in which the food is packed while keeping the inside of the package under a vacuum state, oxygen-free packaging in which an oxygen absorber is used in a package, and gas flush packaging in which a package is sealed in the atmosphere of a desired gas.

In the case of vacuum packaging, for example, rotting of the food such as oxidization with oxygen can be prevented because the vacuum state is kept in the package. Whether the inside of the package is kept under vacuum or not can be relatively easily evaluated by visual assessment of the presence or absence of air flow into the package. Vacuum packaging is also advantageous in terms of storage and display spaces and often used where relatively long-term storage is required. However, because the inside of the package is kept under vacuum, the food is brought into close contact with a film or the like by the atmospheric pressure. Thus, problems arise in terms of displaying such that a product cannot be voluminous, the food is distorted, and so on.

On the other hand, the method in which an oxygen absorber is used in the package, the method in which the food is packaged with a packaging material having an oxygen absorbing layer, and the method of gas flush packaging in which the package is sealed in the atmosphere of a desired gas, are capable of displaying the food in its original shape without crushing the food under the atmospheric pressure. Therefore, these methods are excellent in that the product can be differentiated by the so-called a display effect such that the product can be made to look tasty. Therefore, oxygen-free packaging by absorption of oxygen and gas flush packaging are mainly conducted for products with relatively short shelf lives in a range of several days to one month.

However, in the method of oxygen-free packaging by absorption of oxygen and the method of sealing the package under the atmosphere of a desired gas filled therein (gas flush packaging), it is difficult to visually evaluate the gas environment in the package to assessing whether a suitable gas atmosphere is maintained in the package. Therefore, a method for assessing whether the gas atmosphere in the package is suitable is searched for. It is desired to develop an oxygen indicator capable of detecting the presence or absence of oxygen having a significant influence especially on rotting of food.

As such an oxygen indicator, for example, JP-A-54-138489 (Patent Document 1) discloses a deoxidization indicator composed of methylene blue, a sugar(s), an alkaline material(s), water and ascorbic acid, in which methylene blue is oxidized by oxygen dissolved in water in the oxygen indicator and turns blue if oxygen exists, and methylene blue is reduced by an alkaline sugar solution and turns colorless if no oxygen exists. Also, for example, JP-A-2001-503358 (Patent Document 2) discloses an oxygen indicator in which a redox-sensitive color-indicating material reacts with oxygen via an appropriately selected catalyst such as an enzyme and changes color. These oxygen indicators are excellent in that the presence or absence of oxygen in a packaging container can be visually checked by color change of methylene blue or other redox-sensitive color-indicating materials, when they are enclosed with the packaging container or attached to an oxygen gas permeable portion on a part of the outside of the packaging container.

However, the oxygen indicator composed of methylene blue, a sugar, an alkaline material and water, represented by Patent Document 1, has a problem such that if carbon dioxide exists in the packaging container, for example, in the case of gas flush packaging in which food is packaged using a mixed gas of inert nitrogen and bacteriostatic carbon dioxide in terms of storage of food, carbon dioxide having a higher solubility in water than oxygen is dissolved in water in the oxygen indicator to cause a change in pH, and therefore the action of reducing methylene blue is lost, resulting in an obscured change in color. Furthermore, in some cases, there is also a problem such that methylene blue may turn blue due to the influence of carbon dioxide even in the oxygen-free state, leading to an erroneous determination that oxygen exists. Further, there is also a problem such that if an alcohol is used in the gas flush packaging in terms of bacteriostatic and bacteriocidal effects, the capability of detecting oxygen is lowered due to the influence of the presence the of the alcohol. The oxygen indicator using methylene blue can be used only when neither carbon dioxide nor an alcohol exists, or only when its concentration is very low. Thus, the oxygen indicator is thus forced to undergo various limitations. Further, there is a problem such that the determination of the presence or absence of oxygen depends on the oxidation and reduction of methylene blue itself, and therefore the sensitivity is so high that it is sometimes difficult to set the threshold of the oxygen concentration causing a change in color and set a color change rate or the like to a given value. Furthermore, there is also a problem such that coloring agents other than methylene blue are hard to be used in terms of stability and weathering resistance. Furthermore, there is also a problem such that because the oxygen indicator contains an alkaline material, it inflicts an injury upon a person who inadvertently ingests it.

The oxygen indicator using an enzyme reaction, described in Patent Document 2, can buffer variations in pH with a buffer solution to reduce an influence on detection of oxygen even in the case of gas flush packaging in which carbon dioxide exists in the package. However, the oxygen indicator has a problem such that since the sensitivity of detection of oxygen is low and oxygen itself is unstable, with the lapse of time, the change in color becomes obscured, so that the oxygen detection capability is lowered and in some cases, the oxygen-free state is erroneously taken even though oxygen enters.

Distributors of daily food in supermarkets, convenience stores and the like try to provide healthy daily food and the like having reduced amounts of food preservatives and the like for seeking tastiness of food materials themselves. However, reduction in food preservatives causes the food to be rotten earlier, thus requiring measures for safety of food. Therefore, gas flush packaging has been tried as means for food preservability without using additives such as food preservatives. The gas flush packaging is the method in which the package is sealed under the atmosphere of nitrogen, argon or the like as inert gas to suppress the oxidative spoilage of food by oxygen. In addition to the gas flush packaging, it is well known that other gas is mixed with inert gas for the purpose of inhibiting the growth of microorganisms and the like and for disinfection, in terms of prevention of microbiological contamination of food. Examples of gas for use in the inhibition of growth of microorganisms and the like and for use in disinfection include carbon dioxide and alcohols in terms of low costs and food safety. Carbon dioxide mainly has a bacteriostatic action for inhibiting the growth of microorganisms, and alcohol mainly has an action of killing microorganisms. Gas compositions, in which inert gas, microorganism growth inhibiting gas such as carbon dioxide and microorganism killing gas such as alcohols are mixed, are recently often used in the gas flush packaging, in terms of food safety. Accordingly, an oxygen indicator capable of being used in such a mixed composition of inert gas, microorganism growth inhibiting gas such as carbon dioxide and microorganism killing gas such as alcohols is desired.
Patent Document 1: JP-A-54-138489
Patent Document 2: JP-A-2001-503358

### DISCLOSURE OF THE INVENTION

An object of the present invention is to provide an oxygen indicator capable of detecting the presence or absence of oxygen definitely and stably with high sensitivity over a long term even in the presence of carbon dioxide or alcohol. Another object of the present invention is to provide a package provided with an oxygen indicator using an enzyme(s), having a controlled gas composition in a container or bag, thus making it possible to definitely detect the presence or absence of oxygen even in the presence of carbon dioxide or alcohol.

As a result of conducting vigorous studies for achieving the above objects, the present inventors have completed the present invention. Specifically, the present invention is as follows.
(1) An oxygen indicator using an optical absorption spectral change reaction caused by a substrate in presence of oxygen via an enzymatic catalysis, which comprises an oxygen sensitive solution containing at least a coloring substrate as the substrate, an oxidoreductase, and a reducing agent capable of reducing the oxidized coloring substrate.
(2) An oxygen indicator using an optical absorption spectral change reaction caused by a substrate in presence of oxygen via an enzymatic catalysis, which comprises an oxygen sensitive solution containing at least a coloring substrate as the substrate, an oxidoreductase and an enzyme stabilizer.
(3) An oxygen indicator using an optical absorption spectral change reaction caused by a substrate in presence of oxygen via an enzymatic catalysis, comprises an oxygen sensitive solution containing at least a coloring substrate as the substrate, an oxidoreductase, an enzyme stabilizer, and a reducing agent capable of reducing the oxidized coloring substrate.
(4) The oxygen indicator according to the item (1) or (3), wherein the above described reducing agent is a mercapto group containing compound capable of producing a disulfide group when it is oxidized.
(5) The oxygen indicator according to the item (2) or (3), wherein the above described enzyme stabilizer is a nonionic compound with a surface tension in a 0.2 wt% aqueous solution thereof equal to or less than 0.06 N/m.
(6) The oxygen indicator according to the item (5), wherein the above described nonionic compound is a water-soluble polymer.
(7) The oxygen indicator according to the item (6), wherein the above described water-soluble polymer is a water-soluble polyvinyl alcohol, water-soluble polyglycerin or water-soluble cellulose derivative.
(8) The oxygen indicator according to any one of the items (5) to (7), wherein the oxidoreductase is ascorbate oxidase or bilirubin oxidase.
(9) The oxygen indicator according to any one of the items (1) to (8), wherein the above described oxygen sensitive solution contains a buffer agent.
(10) The oxygen indicator according to any one of the items (1) to (9), wherein the above described oxygen sensitive solution further contains a compound capable of reacting with oxygen in competition with the above described optical absorption spectral change reaction, or a compound capable of adsorbing oxygen.
(11) A package comprising a container or bag, wherein the container or bag contains the oxygen indicator according to any one of the items (1) to (10), or the oxygen indicator according to any one of the items (1) to (10) is mounted in such a manner as to block the opening of the container or bag, whereby the concentration of oxygen in the container or bag can be detected.
(12) The package according to the item (11), wherein the package has a form of vacuum packaging.
(13) The package according to the item (11), wherein the package has a form of gas flush packaging with the above described container or bag filled with a gas containing no oxygen.

The oxygen indicator of the present invention can detect the presence or absence of oxygen by a change in color or the like, definitely and stably with high sensitivity over a long term even in the presence of carbon dioxide or alcohols with gas flush packing, and control the gas composition in the container or bag, thus making it possible to definitely detect the presence or absence of oxygen in the package even in the presence of carbon dioxide or alcohols.

### BRIEF DESCRIPTION OF THE DRAWINGS

FIG. 1 is a conceptual perspective view showing an example of production of an oxygen indicator of the present invention, and an A-A' sectional view thereof;
FIG. 2 is a conceptual perspective view showing an example of production of the oxygen indicator of the present invention, and a B-B' sectional view thereof;
FIG. 3 is a conceptual perspective view showing an example of production of the oxygen indicator of the present invention, and a C-C' sectional view thereof;
FIG. 4 is a conceptual perspective view showing an example of production of the oxygen indicator of the present invention, and a D-D' sectional view thereof;
FIG. 5 is a conceptual perspective view showing an example of production of the oxygen indicator of the present invention, and an E-E' sectional view thereof; and
FIG. 6 is a conceptual perspective view showing an example of use of the oxygen indicator illustrated in FIG. 5.

### BEST MODE FOR CARRYING OUT THE INVENTION

The present invention, particularly preferred embodiments thereof, will be described specifically below.

An oxygen indicator of the present invention is comprised of an oxygen sensitive solution containing a coloring substrate, an oxidoreductase and a specific reducing agent, or an oxygen sensitive solution containing a coloring substrate, an oxidoreductase and an enzyme stabilizer, or an oxygen sensitive solution containing a coloring substrate, an oxidoreductase, an enzyme stabilizer and a reducing agent. The oxygen indicator of the present invention makes a determination on the presence or absence of oxygen with a desired oxygen concentration as a threshold by a combination of those components. Specifically, it detects an increase in oxygen from an oxygen-free state or low oxygen state by a change in color. The oxygen sensitive solution described in the present invention refers to a solution having its color or the like changed such that the dissolved coloring substrate is oxidized with oxygen existing in the atmosphere via an enzymatic catalysis to change the optical absorption spectrum.

The most significant point distinguishing the present invention from the prior art in Patent Document 1 is that the detection of oxygen is not influenced even if carbon dioxide exists in the package. Specifically, the oxygen indicator in Patent Document 1 contains methylene blue, a sugar and an alkaline material, wherein the sugar and the alkaline material prevent methylene blue as a coloring agent from having an oxidized form (coloring state) with oxygen dissolved in the solution, by their reducing action, and make the methylene blue have a colorless reduced form. Therefore, if carbon dioxide becoming acidic when dissolved in water exists, it lowers the reducing action and influences the detection of oxygen.

On the other hand, the present invention is characterized in that the coloring substrate is oxidized with oxygen dissolved in the solution using the catalytic action of the oxidoreductase and that the optical absorption spectrum is changed. Therefore, even if carbon dioxide is dissolved in the solution, an enzyme reaction for making determination on the presence or absence of oxygen or a reaction for reducing the coloring substrate oxidized with the reducing agent is not influenced.

Another point distinguishing the present invention from the prior art in Patent Document 1 is that in the present invention, by selecting a combination of the oxidoreductase and the coloring substrate to be used in every way, the presence or absence of oxygen can be detected with a desired change in the optical absorption spectrum. Moreover, since the enzyme reaction has high substrate selectivity, several types of enzymes and substrates can be used in a mixed state depending on the combination of the enzymes and the substrates to be used. For example, if several kinds of substrates having utterly different oxygen concentrations required for the enzyme reaction, reaction rates and colors during the reaction are used in a mixed state for one kind of enzyme, the color can be stepwise changed according to the oxygen concentration, e.g. yellow in a certain oxygen concentration and blue in a higher oxygen concentration. Also, the color can be stepwise changed according to oxygen exposure time, e.g. brown in a short oxygen exposure time, and red in a long oxygen exposure time.

Further, still another point distinguishing the present invention from the prior art in Patent Document 1 is that if a reducing agent capable of reducing the oxidized coloring substrate is made to coexist, the concentration of the reducing agent is adjusted or the concentrations of the coloring substrate and the oxidoreductase are adjusted, whereby the threshold of the concentration of oxygen to be detected, the rate of change in the optical absorption spectrum, and the like can be set to the desired values.

The most significant point distinguishing the present invention from the prior art in Patent Document 2 is that in the prior art, the stability of the enzyme itself is not considered at all and the oxygen detection performance is easily deteriorated with the lapse of time, while the present invention is characterized in that a specified reducing agent is made to coexist, or an enzyme stabilizer is made to coexist, for stabilizing the enzyme itself, and as a result, the enzyme is prevented from being rapidly inactivated, thus making it possible to make the optical absorption spectral change reaction by the coloring substrate proceed with stability over a long term. The specified reducing agent described herein refers to a mercapto group containing compound capable of producing a disulfide group when oxidized. Among general reducing agents described below, the compound acts especially as an enzyme stabilizer or as an activator depending on the enzyme, and is used for maintaining a stable oxygen detection capability over a long term as an oxygen indictor in the present invention.

The oxidoreductase for use in the present invention is selected from the EC1 group and exhibits a catalytic action for a reaction through which a substrate other than oxygen is chemically changed in the presence of oxygen or a catalytic action in a reaction between a product by an enzymic or non-enzymic reaction and the coloring substrate.

For the former oxidoreductase, there is used an enzyme exhibiting a catalytic action in a reaction system in which the coloring substrate used as a substrate other than oxygen is oxidized or a reaction system in which oxygen is chemically changed into a product such as hydrogen peroxide without using such a coloring substrate.

Examples of the former oxidoreductase include oxidase, flavin monooxygenase, copper hydromonooxygenase, iron monooxygenase, ribulose diphosphate oxygenase, dioxygenase and the like. Preferable specific examples include catechol oxidase (EC1.10.3.1), laccase (EC1.10.3.2), bilirubin oxidase (EC1.3.3.5), ascorbate oxidase (EC1.10.3.3), 3-hydroxyanthranilate oxidase (EC1.10.3.5), alcohol oxidase (EC.1.1.3.13), cholesterol oxidase (EC1.1.3.6) and glucose oxidase (EC1.1.3.4).

The latter oxidoreductases include, for example, peroxidase and the like. As specific examples of the reaction with this enzyme, for example, the coloring substrate undergoes a color reaction by the catalytic action of peroxidase (EC1.11.1.7) using hydrogen peroxide produced by an enzymic or non-enzymic reaction, or a combination of a hydrogen donor and a chromogen as the coloring substrate is subjected to coupling to undergo a color reaction. Furthermore, these color reactions are not specifically limited to the above. For example, the peroxidase coupled color reaction described in "Enzyme Measurement Method written by Takasaka, p. 49-55, Igaku-Shoin (1982)" is used.

In the present invention, according to the combination with the coloring substrate to be used, the oxidoreductase(s) may be appropriately selected from the above-mentioned examples and used alone or in combination of two or more. Of the oxidoreductases described above, bilirubin oxidase (EC1.3.3.5) and ascorbate oxidase (EC1.10.3.3) are more preferable in terms of versality and costs, and ascorbate oxidase originated from Acremonium species is most preferable in terms of enzyme stability.

The concentration of the oxidoreductase in the oxygen sensitive solution is preferably in the range of 0.01 µg/ml to 100 mg/ml irrespective of whether the oxidoreductase is used alone or in combination or two or more. Generally, when the oxidoreductase is dissolved in water, the diluter the solution, the more likely the enzyme activity is declined. The oxidoreductase is expensive as compared to other materials. Thus, as long as the concentration is in this range in the present invention, relatively stable preservability can be ensured and a cost-related problem is not significant, though it depends on the oxidoreductase that is used. The concentration is selected as appropriate from the range described above for the purpose of adjusting the type and nature of the oxidoreductase that is used, the combination with the concentrations of other materials such as the coloring substrate that are used, the activity of the oxidoreductase that is used as a material, or the threshold of the concentration of oxygen to be detected and time required for the detection when used as an oxygen indicator. The concentration is more preferably in the range of 1 to 1000 µg/ml in terms of preservation stability of the enzyme itself and costs.

The coloring substrate in the present invention refers to a compound that has its optical absorption spectrum changed by the reaction of the enzyme as a substrate other than oxygen and is used for detection of oxygen.

The coloring described in the present invention refers to a change in the optical absorption spectrum of a material, and means that the coloring substrate is oxidized by the catalytic action of the oxidoreductase to undergo a change in the chemical structure and nature, resulting in a change of the optical wavelength absorption region. The available optical absorption spectrum may be of any wavelength region as long as the changed wavelength can be measured or detected. For example, a change in optical absorption spectrum in the UV region may be detected using a UV measuring apparatus or the like. A change in the visible ray region (400 nm to 600 nm) may be visually identified without using an apparatus for measuring the wavelength absorbance.

The optical absorption spectral change reaction described in the present invention refers to a reaction through which the coloring substrate changes its optical absorption spectrum via the catalytic action of the oxidation and reduction enzyme in the presence of oxygen.

Changes in chemical structure and nature of the coloring substrate undergoing the optical absorption spectral change include, as specific examples, various changes such as abstracting of hydrogen from a hydroxyl group, amino group or the like, formation of double bonds, association and coupling of substrates, and delocalization of electric charges associated with movement of electrons. In the present invention, the presence or absence of oxygen can be detected with a desired color by selecting the coloring substrate to be used from various kinds. Such coloring substrates are preferably compounds having functional groups of relatively high activity such as a hydroxyl group, an amino group, a cyano group and a carbonyl group, and phenol derivatives, aniline derivatives, toluidine derivatives and benzoic acid derivatives as oxidation-reduction indicators and oxidation-reduction reagents. Specific examples thereof include hydroquinone, polyphenol, p-phenylenediamine, a cyanine dye, aminophenol, N,N-dimethylaniline, N,N-diethylaniline, 2,4-dichlorophenol, N-ethyl-N-(2-hydroxy-3-sulfopropyl)-3,5-dimethoxyaniline (DAOS), N-ethyl-N-sulfopropyl-3,5-dimethylaniline (MAPS), N-ethyl-N-(2-hydroxy-3-sulfopropyl)-3,5-dimethylaniline(MAOS), N-ethyl-N-(2-hydroxy-3-sulfopropyl)-m-toluidine (TOOS), N-ethyl-N-sulfopropyl-m-anisidine (ADPS), N-ethyl-N-sulfopropylaniline (ALPS), N-ethyl-N-sulfopropyl-3,5-dimethoxyaniline (DAPS), N-sulfopropyl-3,5-dimethoxyaniline (HDAPS), N-ethyl-N-sulfopropyl-m-toluidine (TOPS), N-ethyl-N-(2-hydroxy-3-sulfopropyl)-m-anisidine (ADOS), N-ethyl-N-(2-hydroxy-3-sulfopropyl)aniline (ALOS), N-(2-hydroxy-3-sulfopropyl)-3,5-dimethoxyaniline (HDAOS), N-sulfopropyl-aniline (HALPS), o-dianisidine, o-tolidine, 3,3-diaminobenzidine, 3,3,5,5-tetramethylbenzidine, N-(carboxymethylaminocarbonyl)-4,4-bis(dimethylamino)biphenylamine (DA64), 10-(carboxymethylaminocarbonyl)-3,7-bis(dimethylamino)phenothiazine (DA67), 3,5-dinitrobenzoic acid, 5-aminosalicylic acid, 3-hydroxyanthranilic acid, 3,5-diaminobenzoic acid or the like, 4-aminoantipyrine, o-phenylenediamine, 1-amino-2-naphthol-4-sulfonic acid, 1-phenyl-3-methyl-5-pyrazolone, 2-amino-8-naphthol-6-sulfonic acid, 3-methyl-2-benzothiazolinonehydrazone, 2-amino-phenol-4-sulfonic acid, 2,6-dibromo-4-aminophenol, 2,2'-azinol (3-ethylbenzothiazolin-6-sulfonic acid) diammonium salt, 2,2'-azino-bis(3-ethylbenzothiazoline-6-sulfonic acid) ammonium salt (ABTS), 2,6-dichloroindophenol, catechol, tannin, epicatechin, and epigallocatechin or the like. If it is desired to make a fluorescent observation, compounds capable of emitting fluorescence by oxidization, e.g. homovanillic acid, 4-hydroxyphenyl acetate, tyramine, paracresol and diacetylfluorescin derivatives may be mentioned. If it is desired to make a chemiluminescent observation, pyrogallol may be mentioned. The substances mentioned here are only of examples, and any substances capable of remarkably promoting fluorescence emitting reaction by the catalytic action of the enzyme, are included as such. Furthermore, a plurality of compounds which are coupled with each other to change the optical absorption spectrum may be used. For example, they include combinations of 4-aminoantipyrin, 2,6-dibrom-4-aminophenol, ABTS and the like with phenol derivatives, aniline derivatives, 4-hydroxybenzoic acid derivatives and the like.

In the present invention, according to the combination with the oxidoreductase and the like to be used, the coloring substrate(s) may be appropriately selected from the above-mentioned examples and used alone or in combination of two or more. Of the above coloring substrates, benzoic acid derivatives, 2,2'-azino-bis (3-ethylbenzothiazoline-6-sulfonic acid) ammonium salts (ABTS), 1,2-dioxybenzene derivatives, hydroquinone derivatives, 1,4-diaminobenzene derivatives and 3-hydroxyanthranilic acid derivatives are preferable in terms of versality, stability of the coloring substrate itself and costs; and 2,2'-azino-bis (3- ethylbenzothiazoline-6-sulfonic acid) ammonium salts (ABTS) are most preferable in terms of handling characteristics such as solubility in water.

The concentration of the coloring substrate in the oxygen sensitive solution is preferably in the range of 0.01 to 1000 mg/ml in total, irrespective of whether the coloring substrate is used alone or in combination of two or more. In the present invention, as long as the concentration is in this range, a change in the optical absorption spectrum can be definitely recognized and a cost-related problem is not significant, depending on the coloring substrate that is used. The concentration is selected as appropriate from the range described above for the purpose of adjusting the type and nature of the coloring substrate that is used, the combination with the concentrations of other materials such as the oxidoreductase that are used, the absorbance coefficient of the oxidoreductase that is used as a material, or the threshold of the concentration of oxygen to be detected, time required for the detection and a change in chrominance such as color when used as an oxygen indicator. The concentration is more preferably in the range of 0.1 to 50 mg/ml in terms of easy recognition of detection of oxygen and costs as the oxygen indicator.

Generally in the biochemical field, the enzyme is recognized to easily decrease in reaction activity by the influences of heat and pH and, depending on the type of enzyme, usually stored under refrigeration in the dried state, not in the state of solution, when it is stored over a long term. However, if the enzyme is used as an oxygen indicator, the enzyme should be in an oxygen sensitive solution in the state of solution upon performing the enzyme detection reaction described above.

In the present invention, particularly, a mercapto group-containing compound capable of producing a disulfide group when oxidized is used as a reducing agent capable of reducing the oxidized coloring substrate, whereby the detection of oxygen can be stably performed over a long term as a commercial product without significant degradation in reaction activity of the enzyme even in the state of solution. Among the general reducing agents, the mercapto group-containing compound has a property of acting particularly as a stabilizer for the enzyme or as an activator depending on the enzyme. Alternatively, in the present invention, the enzyme stabilizer may be added to the oxygen sensitive solution, whereby the detection of oxygen can be stably performed over a long term as a commercial product without significant degradation in reaction activity of the enzyme even in the state of solution. Use of the enzyme stabilizer is more preferable because not only the above specified reducing agents but also general reducing agents can be used.

The specified reducing agent for use in the present invention is a mercapto group-containing compound capable of producing a disulfide group when oxidized. The mercapto group (-SH group) of the compound is oxidized into the disulfide group (-S-S-group) to prevent the active site of the enzyme from being oxidation-deteriorated. Furthermore, by adjusting the concentration of the compound in the oxygen sensitive solution, the presence or absence of oxygen can be determined with a given oxygen concentration used as a threshold. The compounds include, as specific examples, glutathione, cysteine, cysteine derivatives such as N-acetyl cysteine, mercaptoethanol, dithiothreitol and thioglycerol. In the present invention, according to the combination with the oxidoreductase to be used, the reducing agent may be selected from the above specified examples and used alone or in combination of two or more. Of the above reducing agents, glutathione, cysteine hydrochloride, N-acetyl cysteine and thioglycerol are preferable in terms of versality and costs. The concentration of the compound in the oxygen sensitive solution is not specifically limited. When other enzyme stabilizers than the mercapto group-containing compound are used, the preservation stability of the enzyme is not badly affected even if other general reducing agents are used instead of the mercapto group-containing compound. The concentration of the compound may be adjusted according to the concentrations of the oxidoreductase and the coloring substrate that are used for determining the presence or absence of oxygen with a given oxygen concentration as a threshold. The concentration of the compound is preferably 150 mM or less in terms of solubility and costs, and preferably 80 mM or less in terms of ease of preparation of a solution such as pH adjustment of the oxygen sensitive solution.

Furthermore, general reducing agents other than the specified reducing agents described above include, for example, an alkaline material combined with a reducing sugar, potassium ferrocyanide, dithionites, thiosulfates, sulfites, ascorbic acid, erythorbic acid, oxalic acid, malonic acid, metal salts of these organic acids, and other reducing agents described in Patent Document 2 and other documents. In the present invention, according to the combination with the oxidoreductase and the like to be used, the general reducing agent is appropriately selected from the above-mentioned examples and may be used alone or in combination of two or more. Of these reducing agents, ascorbic acid, erythorbic acid, oxalic acid, malonic acid and metal salts of these organic acids are preferable in terms of versality and costs; ascorbic acid, erythorbic acid and metal salts thereof are more preferable in terms of safety and sanitation. The concentration of the above general reducing agent in the oxygen sensitive solution is not specifically limited. If the above specific reducing agent is used as a reducing agent, the above general reducing agent need not be used. The concentration of the above general reducing agent may be adjusted according to the concentrations of the oxidoreductase and the coloring substrate that are used for determining the presence or absence of oxygen with a given oxygen concentration as a threshold. The concentration of the above general reducing agent is preferably 500 mM or less in terms of solubility and costs. The above general reducing agent can be used in combination with the above specific reducing agent as appropriate. This combination is further preferable because it results in the combination of the action of the above specific reducing agent to prevent deterioration of the enzyme activity and the cost advantage of the general reducing agent.

The enzyme stabilizer to be used in the present invention is preferably selected appropriately depending on the enzyme to be used. For ascorbate oxidase, specific examples include a sugar such as mannitol and proteins such as gelatin and bovine serum albumin. For bilirubin oxidase, they are ethylene diamine tetra-acetic acid (EDTA) and aspartic acid. The enzyme stabilizer may be appropriately selected from the above examples and used alone or in combination of two or more. The concentration of the agent in the oxygen sensitive solution is preferably 0.1 mM or greater for effectively exhibiting the enzyme stabilization action, irrespective of whether the enzyme stabilizer is used alone or in combination of two or more. The concentration of the enzyme stabilizer is not specifically limited as for its upper limit but for some enzymes, is preferably 50 mM or less in terms of costs, since the enzyme stabilization action is almost the same even if it is used in a large amount.

The present inventors newly found that particularly by adding as an enzyme stabilizer a nonionic compound with a surface tension in a 0.2 wt% aqueous solution thereof equal to or less than 0.06 N/m, the reaction activity retaining capability could be improved as compared to other enzyme stabilizers, and the oxygen detection capability could be significantly improved. Consequently, as a commercialized oxygen indicator product, the oxygen detection capability can be stabilized over a long term and effectively, and oxygen can be detected more accurately. If the surface tension is 0.06 N/m or less, the oxygen sensitivity of the oxygen sensitive solution containing the nonionic compound is significantly improved for unknown reason. The surface tension is preferably 0.05 N/m or less for further improving the oxygen detection capability. On the other hand, the lower limit of the surface tension is not specifically limited because it is known as a result of vigorous studies by the present inventors that the oxygen sensitivity of the oxygen sensitive solution is more improved if a compound having a lower surface tension is dissolved. The surface tension in the present invention is a value obtained by measuring the surface tension by a DuNouy surface tension balance (ring method) at 23°C using a 0.2 wt% aqueous solution of a nonionic compound as a measurement sample. Furthermore, if an ionic compound such as sodium dodecyl sulfate is used instead of the nonionic compound, the enzyme may be deactivated and no longer function as an oxygen indicator.

The nonionic compound for use in the present invention refers to a compound that is not ionically dissociated in water. Specific examples of the compound include nonionic water-soluble polymers and nonionic surfactants.

Nonionic surfactants include, for example, glycerin derivatives, sucrose, fatty acid esters such as sorbitol, and alcohol adducts. In the present invention, of these nonionic compounds, nonionic water-soluble polymers are more preferable in terms of the enzyme stabilization action.

Nonionic water-soluble polymers for use in the present invention include, as specific examples, water-soluble compounds with a surface tension in a 0.2 wt% aqueous solution thereof equal to or less than 0.06 N/m, selected from polyvinyl alcohols such as vinyl alcohol copolymers and partially saponified polyvinyl alcohols, polyglycerin derivatives, and cellulose derivatives such as methylcellulose, hydroxypropylmethylcellulose and carboxymethylcellulose. Of these water-soluble polymers, partially saponified polyvinyl alcohols and hydroxypropylmethylcellulose are especially preferable in terms of easy handling such as solubility and costs. The function of the oxygen indicator of the present invention is particularly remarkably improved by addition of these water-soluble polymers when ascorbate oxidase is used as the oxidoreductase and ABTS as the coloring substrate.

The nonionic compound that is used as the enzyme stabilizer in the present invention may be appropriately selected from these compounds and used alone or in combination of two or more. The concentration of the compound in the oxygen sensitive solution is preferably 0.01 wt% or greater irrespective of whether the compound is used alone or used in combination of two or more. If the concentration of the compound is 0.01 wt% or greater, the oxygen sensitivity of the oxygen sensitive solution can be improved effectively. To exhibit the enzyme stabilization action more effectively, the concentration of the compound is more preferably 0.03 wt%. The concentration of the compound is not specifically limited as for its upper limit, but because the oxygen sensitivity improving action is almost the same even if a large amount of the compound is used, it is preferably 2% or less in terms of costs.

Either or both of the above reducing agent and/or enzyme stabilizer may be used in the present invention.

In the present invention, when the oxygen sensitive solution further contains a pH buffer agent, a significant change in pH of the solution is suppressed to prevent variations in enzyme activity and make it possible to carry out the detection of oxygen with stability over a long term. The pH buffer agents include, for example, those that are generally used as pH agents, such as acetate buffers, citrate buffers, malate buffers and phosphate buffers, but are not limited thereto. The pH buffer agent suitable for the oxidoreductase to be used may be selected as appropriate. The pH buffer agent may be appropriately selected from the above examples and used alone or in combination of two or more. The concentration of the pH buffer solution in the oxygen sensitive solution may be set as appropriate according to the concentrations of other substances in the enzyme sensitive solution but specifically. It is preferably 10 mM or greater for exhibiting the buffer action of the oxygen sensitive solution effectively, irrespective of whether the pH buffer agent is used alone or in combination of two or more. The upper limit of the concentration of the pH buffer agent is preferably 1 M or less in terms of solubility and costs.

Further, in the present invention, for the purpose of making the oxygen indicator have a performance as an oxygen absorber, for the purpose of adjusting detection time such as delaying detection of oxygen by the oxygen indicator, for the purpose of drastically changing the optical absorption spectral change reaction by the enzyme from a threshold of the oxygen concentration, or for the purpose of adjusting the oxygen detection sensitivity as the oxygen indicator, the oxygen sensitive solution may contain a compound capable of reacting with oxygen in competition with the reaction through which the coloring substrate undergoes a change in the optical absorption spectrum via the catalytic action of the oxidoreductase in the presence of oxygen, or a compound capable of adsorbing oxygen. These competitive compounds include compounds for which the enzyme to be used shows a high substrate selectivity, e.g. ascorbic acid in ascorbate oxidase and bilirubin in bilirubin oxidase, for the enzymic reaction, and include nitrogen monoxide for the non-enzymic reaction. On the other hand, adsorption compounds include hemoglobin, cobalt bivalent complexes, salen complexes and fluorocarbon compounds. The compound is not limited thereto, and may be used alone or in combination of two or more as long as a suitable compound is selected as appropriate according to the purpose. The concentration of the compound in the oxygen sensitive solution may be set according to the concentrations of other substances in the oxygen sensitive solution.

In the present invention, by making an enzyme inhibitor, a substrate analogue, a clathrate compound and the like coexist in addition the compounds described above for adjusting the oxygen detection performance of the oxygen indicator for the above purpose, the optical absorption spectral change reaction may be delayed or reduced in sensitivity. The inhibitor includs, for example, azides, diethyldithiocarbamic acid, thiosulfates, fluorides, cyanides, PCMB, EDTA, and divalent and trivalent metals. The substrate analogue includes compounds selected as appropriate according to the enzyme to be used. The clathrate compound includes cyclodextrin. Those described here are only an example and do not limit the present invention by any means. Types and concentration of the enzymes and substrates to be used may be combined as appropriate according to the purpose in consideration of the enzyme and substrate.

The optical absorption spectral change reaction using the enzyme, referred to in the present invention, is a solution reaction that usually proceeds in a solvent, in which oxygen and the coloring substrate dissolved in the solution undergo an oxidation-reduction reaction in the presence of the enzyme. Any solvent may be used as long as it does not inhibit the above reaction and dissolves oxygen. If the oxygen indicator is used in food packaging, the solvent is preferably water or a mixed solution of water and ethanol containing water as the dominant component (more than 50 wt%) in terms of handling and food sanitation.

The oxygen indicator of the present invention should have a structure preventing the oxygen sensitive solution from contacting oxygen during production and during storage before monitoring of oxygen, i.e. the structure in which the enzyme and the coloring substrate are isolated from oxygen. Specifically, in a low-oxygen state with the oxygen concentration less than 0.05%, preferably an oxygen-free state, the oxygen sensitive solution is packaged with an oxygen gas barrier film and stored and when it is used, the oxygen gas barrier film is removed or broken, whereby the oxygen sensitive solution is made to contact atmospheric oxygen to detect oxygen. Alternatively, each of the enzyme solution and the coloring substrate solution is packaged with the oxygen gas barrier film and stored in a state isolated from oxygen and when it is used, the oxygen gas barrier film is removed or broken, whereby the enzyme solution and the coloring substrate solution are mixed together and made to contact atmospheric oxygen to detect oxygen. At this time, if the oxygen gas barrier film is placed between the oxygen sensitive solution and atmospheric oxygen, oxygen detection time can be controlled by selecting a film having a proper oxygen permeability.

In the present invention, it is more preferable to impregnate or incorporate the oxygen sensitive solution in a carrier and use the same in terms of handling than using the oxygen sensitive solution in the liquid state. Carriers to be used include plastics, metals, ceramics, crystalline cellulose, inorganic particles, gels and papers. Any of them may be used as long as it does not inhibit the above described optical wavelength shift reaction and forms into a solid spontaneously or by processing. Methods for impregnating or incorporating the oxygen sensitive solution in the carrier include, for example, applying the solution to the carrier, coating the surface of the carrier, and dipping the carrier in the solution. A specific structure is such that a plastic, metal, porous molded object made of ceramic, non-woven fabric, paper, woven fabric or the like impregnated with the oxygen sensitive solution, crystalline cellulose such as Avicel (trade name, Asahi Kasei Corporation) or inorganic particles such as diatom earth containing the oxygen sensitive solution and formed into tablets, a gel such as gelatin or agar encloseing the oxygen sensitive solution, or the like is covered with a film or container having a proper oxygen permeability.

In the present invention, if a plastic is used as the above carrier and covering material, a biodegradable plastic is preferably used in consideration of its low combustion calorie during combustion and degradation in the soil. Biodegradable plastics include, for example, polylactic acid, polyglycolic acid, polycaprolactone, polybutyric acid, polyvaleric acid, aliphatic polyesters composed of hydroxycarboxylic acid such as copolymers thereof, aliphatic polyesters composed of condensation polymers of polyvalent alcohols and polyvalent carbonic acids such as ethylene glycol and adipic acid, aliphatic aromatic copolymerized polyesters with aromatic polyvalent compounds copolymerized therewith, and natural polymers such as starches and celluloses, and include those conforming to specifications of biodegradable plastics, for example, specifications defined by Biodegradable Plastic Society in Japan, ASTMD-6400 in the U.S., and DIN V-54900 in Germany.

The oxygen indicator of the present inveniton may be processed into a structure having a shape of a pouch, label, tape, tablet or cap. For example, if confectionary containing oil and fat prone to oxidative spoilage such as butter is packaged in an oxygen-free state, or if processed meat food such as ham is vacuum-packed, a water absorptive paper impregnated with the oxygen sensitive solution of the present invention is covered with an oxygen permeable film to form a pouch-shaped structure, which is put in a package as the oxygen indicator of the present invention. Thus, the presence or absence of oxygen in the package can be detected. Furthermore, if a child or senior person may inadvertently eats the pouch in a container of daily food or luncheon packaged in gas flush packaging, it is preferable that the oxygen indicator processed into an adhesive label form is bonded to the inner side of the container, or bonded in such a manner to block the opening of the container formed for the purpose of filling the packaging container with gas.

The gas flush packaging referred to in the present invention is a packaging technique also called modified atmosphere packaging, gas filling packaging, or controlled atmosphere packaging. Generally, the gas composition in the container or bag is adjusted as appropriate according to the packaged content. Nitrogen or argon, an inert gas, is usually used as a gas component to substitute the inside of the container or bag. For the purpose of inhibiting the growth of bacteria and fungi, the gas composition in the container or bag is preferably oxygen-free, more preferably contains carbon dioxide having a bacteriostatic action in an amount of 3% or greater, most preferably contains ethanol having a bacteriocidal action in an amount of 0.5% or greater. In beverages, by providing the oxygen indicator of the present invention inside a transparent cap on the top surface, the presence or absence of oxygen can be determined with a change in color even in applications where the conventional oxygen indicator using methylene blue cannot be used as in carbonated drinks containing carbon dioxide.

The oxygen indicator of the present invention may be used in any applications other than the food packaging applications described above as long as the presence or absence of oxygen in a sealed space should be determined. For example, the oxygen indicator of the present invention may be used in packaging of precision machinery parts, packaging of metal parts such as screws, packaging of electric parts such as electronic boards and packaging of pharmaceuticals and cosmetics. The package of the present invention may have any form that is generally used as a packaging material such as a bag-shaped or container-shaped form. The material to be used preferably has gas barrier properties for keeping the package under vacuum or reducing variations in the gas composition in the package to the minimum. Materials of the package include plastics, metals, woods, papers and glass or laminates thereof. For its gas barrier properties, variations in the gas composition in the package are preferably reduced to variations below 10% under standard conditions (23°C, 50% RH) for each gas that is used. The container herein refers to a vessel comprised of a receiving container and a lid and intended to accommodate contents, and may be, for example, the so-called food pack with a container and a lid jointed together at one edge via a hinge portion.

In any case, the oxygen indicator of the present invention is intended for determining the presence or absence of oxygen, and is preferably packaged in gas flush packaging with a material having gas barrier properties for isolating the oxygen indicator from oxygen so that the optical absorption spectral change reaction does not proceed or only slightly proceeds before monitoring (especially during storage). For example, the oxygen indicator is stored in a container having high oxygen barrier properties such as a metal or glass, or with a bag packaging of an oxygen gas barrier film. Furthermore, more preferably, an oxygen trapping agent such as a deoxidizer may be put in the storage bag for trapping a very small amount of oxygen in the storage environment and oxygen entering through the oxygen gas barrier storage bag.

Specific examples of the oxygen indicator of the present invention are described below using the drawings.

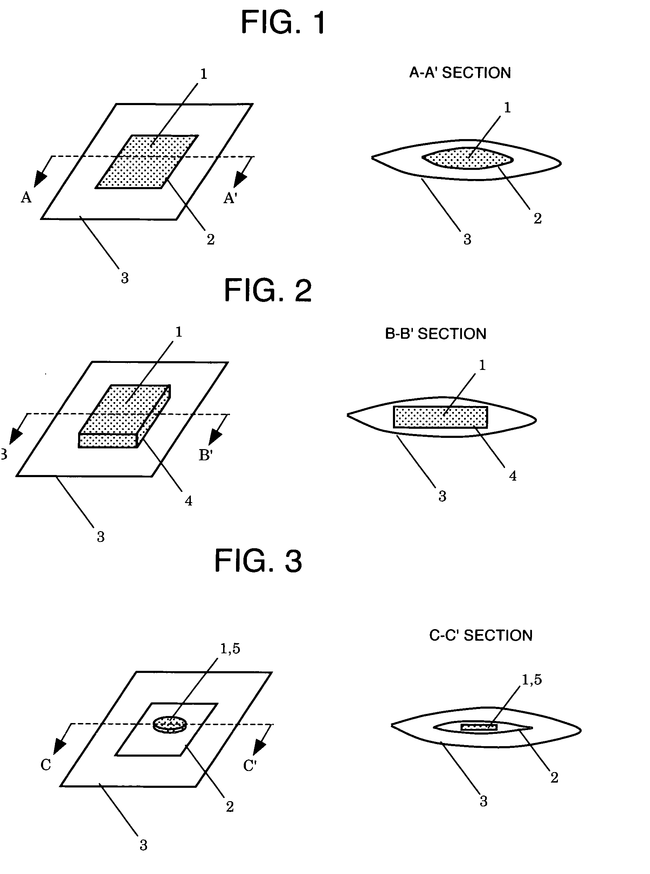

FIG. 1 is a perspective view of an oxygen indicator in which an oxygen sensitive solution 1 is packaged with a bag 2 made of an oxygen gas permeable film in a low oxygen state, and further packaged with a bag 3 made of an oxygen gas barrier film; and a sectional view taken along the A-A' face thereof.

FIG. 2 is a perspective view of an oxygen indicator in which the oxygen sensitive solution 1 is packaged with a plastic container 4 having oxygen permeability in a low oxygen state, and further packaged with the bag 3 made of an oxygen gas barrier film; and a sectional view taken along the B-B' face thereof.

FIG. 3 is a perspective view of an oxygen indicator in which the oxygen sensitive solution 1 is impregnated into a small piece 5 such as a porous molded object, a sheet-shaped body such as a nonwoven fabric, a tablet-molded object using crystalline cellulose or inorganic particles, a gel such as gelatin or agar or a water absorptive filter paper, in a low oxygen state, packaged with the bag 2 made of an oxygen gas permeable film in a low oxygen state, and further packaged with the bag 3 made of an oxygen gas barrier film; and a sectional view taken along the C-C' face thereof.

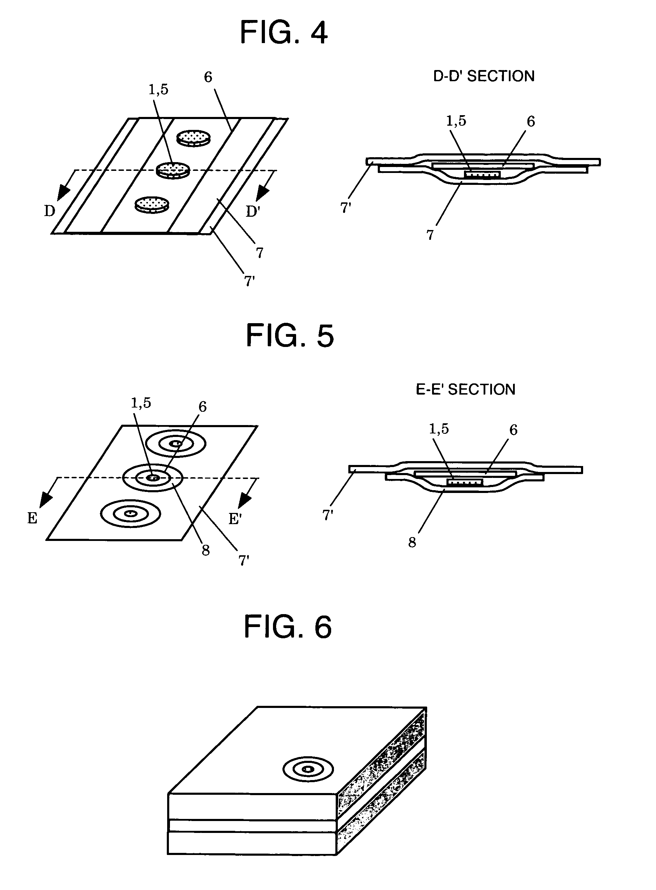

FIG. 4 is a perspective view of an oxygen indicator having the structure in which the filter paper 5 is impregnated with the oxygen sensitive solution 1 in a low oxygen state, the impregnated filter paper 5 is bonded to one adhesive surface of an oxygen gas barrier adhesive tape 7 having adhesive layers on both sides, and the filter paper 5 is covered with an oxygen permeable film 6 from above, bonded together with the adhesive force of the oxygen gas barrier adhesive tape 7, covered with an oxygen gas barrier tape 7' from above the oxygen permeable film 6, and bonded together with the adhesive force of the oxygen gas barrier adhesive tape 7; and a sectional view taken along the D-D' face thereof.

FIG. 5 is a perspective view of an oxygen indicator having the structure in which the filter paper 5 is bonded to the adhesive surface of an oxygen gas barrier adhesive label 8 having an adhesive layer on one side in a low oxygen state and impregnated with the oxygen sensitive solution 1, and the filter paper 5 is covered with the oxygen permeable film 6 from above, bonded together with the adhesive force of the oxygen gas barrier adhesive label 8, covered with the oxygen gas barrier tape 7' from above the oxygen permeable film 6, and bonded together with the adhesive force of the oxygen gas barrier adhesive label 8; and a sectional view taken along the E-E' face thereof.

FIG. 6 is a perspective view showing the case where the adhesive label oxygen indicator shown in FIG. 5 is bonded to a lid having an opening in such a manner to block the opening. In this case, since the opening is blocked with the oxygen gas barrier adhesive label 8, the inside of the container is in a sealed state such that it is insulated from the outer world by a gas barrier material, and variations in the gas composition in the container is thus suppressed. On the other hand, since the indicator has the structure in which the filter paper 5 impregnated with the oxygen sensitive solution 1 is covered with the oxygen permeable film 6, the filter paper 5 contacts the atmosphere in the container via the oxygen permeable film 6, and the concentration of oxygen in the container can be monitored with the indicator.

### Example 1

Bilirubin oxidase (EC1.3.3.5, BO-3 manufactured by Amano Pharmaceuticals Co., Ltd.) was used as an oxidoreductase, ABTS (high quality analysis reagent manufactured by Tokyo Kasei Kogyo Co., Ltd.) was used as a coloring substrate, and a 50 mM phosphate buffer solution with the oxygen concentration of 4 ppm (pH=6.0, prepared from monopotassium phosphate and dipotassium phosphate, reagent chemicals manufactured by Wako Pure Chemical Industries Co., Ltd.) was used as a solvent. Ten micrograms of the enzyme and 3 mg of the substrate were dissolved in 100 µl of phosphate buffer solution to prepare a pre-preparation enzyme solution and a pre-preparation substrate solution, respectively. Further, 2800 µl of phosphate buffer solution, 100 µl of pre-preparation enzyme solution and 100 µl of pre-preparation substrate solution were mixed together and in the mixture, glutathione (reduced form, reagent chemical manufactured by Wako Pure Chemical Industries Co., Ltd.) was dissolved as a reducing agent in a concentration of 0.6 mM to prepare a mixed solution of the enzyme and the substrate. As shown in FIG. 1, this mixed solution (oxygen sensitive solution) 1 of the enzyme and the substrate was packaged with a bag 2 made of an oxygen gas permeable film in a low oxygen state to form an oxygen indicator. It was further packaged with a bag 3 made of an oxygen gas barrier film to form an oxygen indicator. When only the outer bag 3 made of the oxygen gas barrier film was broken in a package to detect oxygen, the oxygen indicator was colorless in an oxygen-free state but turned bluish green when contacting air. Furthermore, the oxygen indicator was transparent in a measurement environment having an oxygen concentration of 1% and turned bluish green under an oxygen concentration of 2%, thus exhibiting sharp coloring characteristics. The oxygen indicator was packaged with an oxygen absorbent in the bag 3 made of the oxygen gas barrier film and stored at 5°C for 10 days after the fabrication of the package. The presence or absence of oxygen was detected in the same manner as described above using the stored oxygen indicatorg. The oxygen indicator was transparent in a measurement environment having an oxygen concentration of 1% and turned bluish green in an oxygen concentration of 2%. There was no difference in oxygen detection capability of the oxygen indicator immediately after the fabrication and after the storage at 5°C for 10 days. It can be understood that the oxygen indicator is excellent in storage stability.

### Example 2

Bilirubin oxidase (EC1.3.3.5, BO-3 manufactured by Amano Pharmaceuticals Co., Ltd.) as an oxidoreductase and polyvinyl alcohol with the saponification degree of 80 mol% (Special Grade reagent chemical manufactured by Wako Pure Chemical Industries Co., Ltd., the surface tension in a 0.2 wt% solution thereof = 0.05 1N/m) as an enzyme stabilizer were dissolved in distilled water together with a previously prepared 200 mM phosphate buffer solution (pH = 6.0, prepared from monopotassium phosphate and dipotassium phosphate, reagent chemicals manufactured by Wako Pure Chemical Industries Co., Ltd.) to prepare an enzyme solution (A1) having 0.35 µg/ml of bilirubin oxidase, 0.01% of polyvinyl alcohol and 50 mM of phosphate buffer solution. ABTS (high quality analysis grade reagent manufactured by Tokyo Kasei Kogyo Co., Ltd.) as a coloring substrate, glutathione (reduced form, Special Grade reagent chemical manufactured by Wako Pure Chemical Industries Co., Ltd.) as a reducing agent, the above polyvinyl alcohol as an enzyme stabilizer were dissolved in distilled water together with a previously prepared 200 mM phosphate buffer solution (pH = 6.0) to prepare a substrate solution (B1) having 0.1 mg/ml of ABTS, 1.2 mM of glutathione, 0.01% of polyvinyl alcohol and 50 mM of phosphate buffer solution. The enzyme solution (A1) and the substrate solution (B1) were each subjected to nitrogen bubbling under a low oxygen environment with the oxygen concentration of 30 ppm so that the concentration of dissolved oxygen was 0.00 mg/L (measured with a dissolved oxygen meter MO 128 manufactured by Mettler-Toledo International Inc.). Then 100 µl of each of the enzyme solution (A1) and the substrate solution (B1) was measured and mixed together to prepare an oxygen sensitive solution (C1). Subsequently, a filter paper (chromatography paper 3MMChr manufactured by Wattman Co., Ltd.) was impregnated with part of the oxygen sensitive solution (C1) in a low oxygen state as shown in FIG. 3, and packaged with a bag made of an oxygen gas permeable film (OPS film (thickness of 25 µm) manufactured by Asahi Kasei Corporation) in a low oxygen state to fabricate an oxygen indicator (D1). Further, the obtained oxygen indicator (D1) was packaged with a bag made of an oxygen gas barrier film (Hiryu Series Standard Bag manufactured by Asahi Kasei Pax) together with an oxygen absorbent in a low oxygen state. Then, using carbon dioxide gas, nitrogen gas and a mixed gas of nitrogen and oxygen, the measurement environment was adjusted so that the oxygen gas component had a predetermined concentration (0.5 vol%, 1.0 vol%, 2 vol%, measured with Check Point manufactured by DANSENSOR Co., Ltd.) with the carbon oxide gas component kept at 50 vol%. The obtained oxygen indicator (D1) was broken only at the outer bag made of the oxygen gas barrier film to detect the presence or absence of oxygen in the measurement environment. The oxygen indicator was transparent in an oxygen concentration of 0.5% in a measurement environment and turned bluish green in a oxygen concentration of 1%. The presence of oxygen was sharply indicated with a threshold of an oxygen concentration of 1%. The oxygen indicator (D1), which was packed together with the oxygen absorbent in a bag made of the oxygen gas barrier film, was stored at 5°C for 30 days after the fabrication, and then tested for oxygen detection in the same way as described above. The results showed again that the oxygen indicator was transparent in an oxygen concentration of 0.5% in the measurement environment and turned bluish green in an oxygen concentration of 1%, thus sharply indicating the presence of oxygen with a threshold of an oxygen concentration of 1%. There was no difference in oxygen detection capability of the oxygen indicator (D1) immediately after the fabrication and after the storage at 5°C for 30 days, and therefore it can be understood that the oxygen indicator is excellent in storage stability.

### Example 3

Bilirubin oxidase (EC1.3.3.5) as an oxidoreductase and hydroxypropylmethylcellulose with the methyl group substitution degree of 1.9 and the hydroxypropylmethyl group substitution degree of 0.25 (Metolose 60SH-15 manufactured by Shin-Etsu Chemical Co., Ltd., the surface tension in 0.2 wt% aqueous solution = 0.047 N/m) as an enzyme stabilizer were dissolved in distilled water together with a previously prepared 200 mM phosphate buffer solution (pH = 6.5) to prepare a 50 mM enzyme solution (A2) having 2.0 µg/ml of bilirubin oxidase, 0.5% of hydroxypropylmethylcellulose and 50 mM of phosphate buffer solution. ABTS as a coloring substrate, N-acetylcysteine (Special Grade reagent chemical manufactured by Wako Pure Chemical Industries Co., Ltd.) as a reducing agent, and the above hydroxypropylmethylcellulose as an enzyme stabilizer were dissolved in distilled water together with a previously prepared 200 mM phosphate buffer solution (pH = 6.5) to prepare a substrate solution (B2) having 3.0 mg/ml of ABTS, 4.0 mM of N-acetylcysteine, 0.5% of hydroxypropylmethylcellulose and 50 mM of phosphate buffer solution. The enzyme solution (A2) and the substrate solution (B2) were each subjected to nitrogen bubbling under a low oxygen environment with the oxygen concentration of 30 ppm so that the concentration of dissolved oxygen was 0.00 mg/L. Then 100 µl of each of the enzyme solution (A2) and the substrate solution (B2) was measured and mixed together to prepare an oxygen sensitive solution (C2). Subsequently, a filter paper was impregnated with part of the oxygen sensitive solution (C2) in a low oxygen state as shown in FIG. 3, and packaged with a bag made of an oxygen gas permeable film in a low oxygen state to fabricate an oxygen indicator (D2). Further, the obtained oxygen indicator (D2) was packaged with a bag made of an oxygen gas barrier film together with an oxygen absorbent in a low oxygen state. Then, the obtained oxygen indicator (D2) was broken only at the outer bag made of the oxygen gas barrier film in the measurement environment in the same manner as in Example 2 to detect the presence or absence of oxygen. The oxygen indicator was transparent in an oxygen concentration of 0.5% in the measurement environment and turned bluish green in an oxygen concentration of 1%, thus sharply indicating the presence of oxygen with a threshold of an oxygen concentration of 1%. The oxygen indicator (D2) was packaged with an oxygen absorbent in a bag made of the oxygen gas barrier film and stored at 5°C for 30 days after the fabrication of the package. The presence or absence of oxygen was detected in the same manner as in Example 2 using the stored oxygen indicator. The oxygen indicator was transparent in an oxygen concentration of 0.5% in the measurement environment and turned bluish green in an oxygen concentration of 1%, thus sharply indicating the presence of oxygen with a threshold of an oxygen concentration of 1%. There was no difference in oxygen detection capability of the oxygen indicator (D2) immediately after the fabrication and after the storage at 5°C for 30 days. It can be understood that the oxygen indicator is excellent in storage stability.

### Example 4

Bilirubin oxidase (EC1.3.3.5) as an oxidoreductase and polyglycerin caprate with the polymerization degree of 10 (Poem C-781 manufactured by Riken Vitamin Co., Ltd., surface tension in 0.2 wt% aqueous solution = 0.057 N/m) as an enzyme stabilizer were dissolved in distilled water together with a previously prepared 400 mM phosphate buffer solution (pH = 5.0) to prepare an enzyme solution (A3) having 20 µg/ml of bilirubin oxidase, 10% of polyglycerin caprate and 100 mM of phosphate buffer solution. ABTS as a coloring substrate, manganese oxalate (dihydrate manufactured by Wako Pure Chemical Industries Co., Ltd.) as a reducing agent, and the above polyglycerin caprate as an enzyme stabilizer were dissolved in distilled water together with a previously prepared 400 mM phosphate buffer solution (pH = 5.0) to prepare a substrate solution (B3) having 1.0 mg/ml of ABTS, 10 mM of manganese oxalate, 10% of polyglycerin caprate and 100 mM of phosphate buffer solution. The enzyme solution (A3) and the substrate solution (B3) were each subjected to nitrogen bubbling under a low oxygen environment with the oxygen concentration of 30 ppm so that the concentration of dissolved oxygen was 0.00 mg/L. Then 100 µl of each of the enzyme solution (A3) and the substrate solution (B3) was measured and mixed together to prepare an oxygen sensitive solution (C3). Subsequently, a filter paper was impregnated with part of the oxygen sensitive solution (C3) in a low oxygen state as shown in FIG. 4. The impregnated filter paper was bonded to one adhesive surface of an oxygen gas barrier adhesive tape (PET manufactured by Sato Seal Co., Ltd., thickness: 75 µm) having adhesive layers on both sides; covered with an oxygen permeable film from above; bonded together with the adhesive force of the oxygen gas barrier adhesive tape; covered with an oxygen gas barrier tape (aluminum laminate film manufactured by Asahi Kasei Pax) from above the oxygen permeable film; and bonded together with the force of the oxygen gas barrier adhesive tape to fabricate an oxygen indicator (D3). Further, the obtained oxygen indicator (D3) was packaged with a bag made of an oxygen gas barrier film together with an oxygen absorbent. Then, in the same manner as in Example 2, the obtained oxygen indicator (D3) was broken at the outer bag made of the oxygen gas barrier film in the measurement environment, and the oxygen gas barrier tape was removed to detect the presence or absence of oxygen. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. The oxygen indicator (D3) was packaged with a bag made of the oxygen gas barrier film together with the oxygen absorbent and then stored at 5°C for 30 days after the fabrication. The presence or absence of oxygen was detected in the same manner as in Example 2 using the stored oxygen indicator. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. There was no difference in oxygen detection capability of the oxygen indicator (D3) immediately after the fabrication and after the storage at 5°C for 30 days. It can be understood that the oxygen indicator is excellent in storage stability.

### Example 5

Ascorbate oxidase (EC1.10.3.3, ASOM manufactured by Asahi Kasei Corporation) as an oxidoreductase and methylcellulose with the methyl group substitution degree of 1.8 (Metolose SM-15 manufactured by Shin-Etsu Chemical Co., Ltd., surface tension in 0.2 wt% aqueous solution = 0.054 N/m) as an enzyme stabilizer were dissolved in distilled water together with a previously prepared 400 mM citrate buffer solution (pH = 4.0, prepared from citric acid and sodium citrate, Special Grade reagent chemicals manufactured by Waco Pure chemical Industries Co., Ltd.) to prepare an enzyme solution (A4) having 10 µg/ml of ascorbate oxidase, 2.0% of methylcellulose and 50 mM of citrate buffer solution. ABTS as a coloring substrate, cysteine hydrochloride (Grade 1 reagent chemical manufactured by Waco Pure chemical Industries Co., Ltd.) as a reducing agent, and the above methylcellulose as an enzyme stabilizer were dissolved in stilled water together with a previously prepared 400 mM citrate buffer solution (pH = 4.0) to prepare a substrate solution (B4) having 8.0 mg/ml of ABTS, 10 mM of cysteine hydrochloride, 2.0% of methylcellulose and 50 mM of citrate buffer solution. The enzyme solution (A4) and the substrate solution (B4) were each subjected to nitrogen bubbling under a low oxygen environment with the oxygen concentration of 30 ppm so that the concentration of dissolved oxygen was 0.00 mg/L. Then 100 µl of each of the enzyme solution (A4) and the substrate solution (B4) was measured and mixed together to prepare an oxygen sensitive solution (C4). Subsequently, a filter paper was impregnated with part of the oxygen sensitive solution (C4) in a low oxygen state as shown in FIG. 4; the impregnated filter paper was bonded to one adhesive surface of an oxygen gas barrier adhesive tape having adhesive layers on both sides; covered with an oxygen permeable film from above; bonded together with the adhesive force of the oxygen gas barrier adhesive tape; covered with an oxygen gas barrier tape from above the oxygen permeable film; and bonded together with the force of the oxygen gas barrier adhesive tape to fabricate an oxygen indicator (D4). Further, the obtained oxygen indicator (D4) was packaged with a bag made of an oxygen gas barrier film together with an oxygen absorbent. Then, in the same manner as in Example 2, the obtained oxygen indicator (D4) was broken at the outer bag made of the oxygen gas barrier film in the measurement environment. The oxygen gas barrier tape was removed to detect the presence or absence of oxygen. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. The oxygen indicator (D4) was packaged with a bag made of the oxygen gas barrier film together with the oxygen absorbent and then stored at 5°C for 30 days after the fabrication. The presence or absence of oxygen was detected in the same manner as in Example 2 using the stored oxygen indicator. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. There was no difference in oxygen detection capability of the oxygen indicator (D4) immediately after the fabrication and after the storage at 5°C for 30 days. It can be understood that the oxygen indicator is excellent in storage stability.

### Example 6

Ascorbate oxidase (EC1.10.3.3) as an oxidoreductase was dissolved in distilled water to prepare a 5 mg/ml ascorbate oxidase mother liquor. Polyvinyl alcohol with the saponification degree of 80 mol% (Special Grade reagent chemical manufactured by Wako Pure Chemical Co., Ltd., surface tension in 2 wt% aqueous solution = 0.051 N/m) as an enzyme stabilizer was dissolved in distilled water to a 1 wt% polyvinyl alcohol mother liquid. The ascorbate oxidase mother liquid and the polyvinyl alcohol mother liquid were dissolved in distilled water together with a previously prepared 400 mM acetate buffer solution (pH = 4.5, prepared from acetic acid and sodium acetate, Special Grade reagent chemicals manufactured by Wako Pure Chemical Industries Co., Ltd.) to prepare 100 ml of enzyme solution (A5) having 100 µg/ml of ascorbate oxidase, 0.05% of polyvinyl alcohol and 100 mM of acetate buffer solution. ABTS as a coloring substrate was dissolved in distilled water to prepare a 25 mg/ml ABTS mother liquid. L-ascorbic acid (Special Grade reagent chemical manufactured by Wako Pure Chemical Industries Co., Ltd.) as a reducing agent was dissolved in distilled water to prepare a 100 mM L-ascorbic acid mother liquid. In the same manner as described above, the above polyvinyl alcohol as an enzyme stabilizer was dissolved in distilled water to prepare a 1 wt% polyvinyl alcohol mother liquid. The ABTS mother liquid, the L-ascorbic acid mother liquid and the polyvinyl alcohol mother liquid were dissolved in distilled water together with a previously prepared 400 mM acetate buffer solution (pH = 4.5) to prepare 100 ml of enzyme solution (B5) having 8.0 mg/ml of ABTS, 25 mM of L-ascorbic acid, 0.05% of polyvinyl alcohol and 100 mM of acetate buffer solution. The enzyme solution (A5) and the substrate solution (B5) were each subjected to nitrogen bubbling under an airtight circumstance in a container with a check valve so that the concentration of dissolved oxygen was 0.00 mg/L. Then each of the solutions was fed to a mixer in an equal amount by a micro pump to continuously prepare an oxygen sensitive solution (C5) with the enzyme solution (A5) and the substrate solution (B5) mixed together. A filter paper bonded to the adhesive surface of an oxygen gas barrier adhesive label (PET manufactured by Sato Seal Co., Ltd., thickness: 75 µm) having an adhesive layer on one side as shown in FIG. 5 was impregnated with part of the oxygen sensitive solution (C5) under a low oxygen environment with the oxygen concentration of 30 ppm, and the filter paper was covered with an oxygen permeable film (OPS film manufactured by Asahi Kasei Corporation, thickness: 25 µm) from above, bonded together with the adhesive force of the oxygen gas barrier adhesive label, covered with an oxygen gas barrier tape from above the oxygen permeable film, and bonded together with the adhesive force of the oxygen gas barrier adhesive label to fabricate an oxygen indicator (D5). Further, the obtained oxygen indicator (D5) was packaged with a bag made of an oxygen gas barrier film together with an oxygen absorbent. Then, in the same manner as in Example 2, the obtained oxygen indicator (D5) was broken at the outer bag made of the oxygen gas barrier film in the measurement environment. The oxygen gas barrier tape was removed to detect the presence or absence of oxygen. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. The oxygen indicator 5 was packaged with a bag made of the oxygen gas barrier film together with the oxygen absorbent and then stored at 5°C for 30 days after the fabrication. The presence or absence of oxygen was detected in the same manner as in Example 2 using the stored oxygen indicator. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. There was no difference in oxygen detection capability of the oxygen indicator (D5) immediately after the fabrication and after the storage at 5°C for 30 days. It can be understood that the oxygen indicator is excellent in storage stability.

### Example 7

Ascorbate oxidase (EC1.10.3.3) as an oxidoreductase was dissolved in distilled water to prepare a 5 mg/ml ascorbate oxidase mother liquid. Hydroxypropylmethylcellulose with the methyl group substitution degree of 1.9 and the hydroxypropylmethyl group substitution degree of 0.25 (Metolose 60SH-15 manufactured by Shin-Etsu Chemical Co., Ltd., surface tension in 0.2 wt% aqueous solution = 0.047 N/m) as an enzyme stabilizer was dissolved in distilled water to prepare a 2 wt% hydroxypropylmethylcellulose mother liquid. The ascorbate oxidase mother liquid and the hydroxypropylmethylcellulose mother liquid were dissolved in distilled water together with a previously prepared 1 M acetate buffer solution (pH = 3.5) to prepare 100 ml of an enzyme solution (A6) having 200 µg/ml of ascorbate oxidase, 0.1% of hydroxypropylmethylcellulose and 200 mM of acetate buffer solution. ABTS as a coloring substrate was dissolved in distilled water to prepare a 25 mg/ml ABTS mother liquid. Sodium L-ascorbate as a first reducing agent was dissolved in distilled water to prepare a 500 mM sodium L-ascorbate mother liquid. N-acetylcysteine as a second reducing agent was dissolved in distilled water to prepare a 200 mM N-acetylcysteine mother liquid. In the same manner as described above, the above hydroxypropylmethylcellulose as an enzyme stabilizer was dissolved in distilled water to prepare a 2 wt% hydroxypropylmethylcellulose mother liquid. The ABTS mother liquid, the sodium L-ascorbate mother liquid, the N-acetylcysteine mother liquid and the hydroxypropylmethylcellulose mother liquid were dissolved in distilled water together with a previously prepared 1 M acetate buffer solution (pH = 3.5) to prepare 100 ml of substrate solution (B6) having 4.0 mg/ml of ABTS, 200 mM of sodium L-ascorbate, 80 mM of N-acetylcysteine, 0.1% of hydroxypropylmethylcellulose and 200 mM of acetate buffer solution. The enzyme solution (A6) and the substrate solution (B6) were each subjected to nitrogen bubbling under an airtight circumstance in a container with a check valve so that the concentration of dissolved oxygen was 0.00 mg/L. Then each of the solutions was fed to a mixer in an equal amount by a micro pump to continuously prepare an oxygen sensitive solution (C6) with the enzyme solution (A6) and the substrate solution (B6) mixed together. A filter paper bonded to the adhesive surface of an oxygen gas barrier adhesive label having an adhesive layer on one side as shown in FIG. 5 was impregnated with part of the oxygen sensitive solution (C6) under a low oxygen environment with the oxygen concentration of 30 ppm. The filter paper was covered with an oxygen permeable film from above; bonded together with the adhesive force of the oxygen gas barrier adhesive label; covered with an oxygen gas barrier tape from above the oxygen permeable film; and bonded together with the adhesive force of the oxygen gas barrier adhesive label to fabricate an oxygen indicator (D6). The oxygen indicator (D6) taken out to the atmosphere was packaged in nitrogen gas flush with a bag made of an oxygen gas barrier film together with an oxygen absorbent. Then, in the same manner as in Example 2, the obtained oxygen indicator (D6) was broken at the outer bag made of the oxygen gas barrier film in the measurement environment. The oxygen gas barrier tape was removed to detect the presence or absence of oxygen. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. The oxygen indicator (D6) was packaged with a bag made of the oxygen gas barrier film together with the oxygen absorbent and then stored at 5°C for 30 days after the fabrication. The presence or absence of oxygen was detected in the same manner as in Example 2 using the stored oxygen indicator. The oxygen indicator was transparent in an oxygen concentration of 0.5% in the measurement environment and turned bluish green in an oxygen concentration of 1%, thus sharply indicating the presence of oxygen with a threshold of the oxygen concentration of 1%. There was no difference in oxygen detection capability of the oxygen indicator (D6) immediately after the fabrication and after the storage at 5°C for 30 days. It can be understood that the oxygen indicator is excellent in storage stability.

### Example 8

Ascorbate oxidase (EC1.10.3.3) as an oxidoreductase was dissolved in distilled water to prepare a 5 mg/ml ascorbate oxidase mother liquid. Without adding an enzyme stabilizer, the ascorbate oxidase was dissolved in distilled water together with a previously prepared 400 mM acetate buffer solution (pH = 4.5) to prepare 100 ml of an enzyme solution (A7) having 100 µg/ml of ascorbate oxidase and 100 mM of acetate buffer solution. ABTS as a coloring substrate was dissolved in distilled water to prepare a 25 mg/ml ABTS mother liquid. L-ascorbic acid as a reducing agent was dissolved in distilled water to prepare a 100 mM L-ascorbic acid mother liquid. Without adding an enzyme stabilizer, the ABTS mother liquid and the L-ascorbic acid mother liquid were dissolved in distilled water together with a previously prepared 400 mM acetate buffer solution (pH = 4.5) to prepare 100 ml of a substrate solution (B7) having 8.0 mg/ml of ABTS, 25 mM of L-ascorbic acid and 100 mM of acetate buffer solution. The enzyme solution (A7) and the substrate solution (B7) were each subjected to nitrogen bubbling under an airtight circumstance in a container with a check valve so that the concentration of dissolved oxygen was 0.00 mg/L. Then each of the solutions was fed to a mixer in an equal amount by a micro pump to continuously prepare an oxygen sensitive solution (C7) with the enzyme solution (A7) and the substrate solution (B7) mixed together. A filter paper bonded to the adhesive surface of an oxygen gas barrier adhesive label (PET manufactured by Sato Seal Co., Ltd., thickness: 75 µm) having an adhesive layer on one side as shown in FIG. 5 was impregnated with part of the oxygen sensitive solution (C7) under a low oxygen environment with the oxygen concentration of 30 ppm. The filter paper was covered with an oxygen permeable film (OPS film manufactured by Asahi Kasei Corporation, thickness: 25 µm) from above; bonded together with the adhesive force of the oxygen gas barrier adhesive label; covered with an oxygen gas barrier tape from above the oxygen permeable film; and bonded together with the adhesive force of the oxygen gas barrier adhesive label to fabricate an oxygen indicator (D7). Further, the obtained oxygen indicator (D7) was packaged with a bag made of an oxygen gas barrier film together with an oxygen absorbent. Then, in the same manner as in Example 2, the obtained oxygen indicator (D7) was broken at the outer bag made of the oxygen gas barrier film in the measurement environment. The oxygen gas barrier tape was removed to detect the presence or absence of oxygen. The oxygen indicator was transparent in oxygen concentrations of 0.5% and 1% in the measurement environment and turned bluish green in an oxygen concentration of 2%, thus indicating the presence of oxygen with a threshold of the oxygen concentration of 2%. The oxygen indicator (D7) was packaged with a bag made of the oxygen gas barrier film together with the oxygen absorbent and then stored at 5°C for 10 days after the fabrication. The presence or absence of oxygen was detected in the same manner as in Example 2 using the stored oxygen indicator. The oxygen indicator was transparent in oxygen concentrations of 0.5% and 1% in the measurement environment and turned only slightly bluish green in an oxygen concentration of 2%. There was a definite difference in oxygen detection capability of the oxygen indicator (D7) immediately after the fabrication and after the storage at 5°C for 10 days.

### Example 9

Ascorbate oxidase (EC1.10.3.3) as an oxidoreductase was dissolved in distilled water to prepare a 5 mg/ml ascorbate oxidase mother liquid. Polyvinyl alcohol with the saponification degree of 80 mol% (Special Grade reagent chemical manufactured by Wako Pure Chemical Co., Ltd., surface tension in 2 wt% aqueous solution = 0.051 N/m) as an enzyme stabilizer was dissolved in distilled water to prepare a 1 wt% polyvinyl alcohol mother liquid. The ascorbate oxidase mother liquid and the polyvinyl alcohol mother liquid were dissolved in distilled water together with a previously prepared 400 mM acetate buffer solution (pH = 4.5, prepared from acetic acid and sodium acetate, Special Grade reagent chemicals manufactured by Wako Pure Chemical Industries Co., Ltd.) to prepare 100 ml of an enzyme solution (A8) having 100 µg/ml of ascorbate oxidase, 0.05% of polyvinyl alcohol and 100 mM of acetate buffer solution. ABTS as a coloring substrate was dissolved in distilled water to prepare a 25 mg/ml ABTS mother liquid. In the same manner as described above, the above polyvinyl alcohol as an enzyme stabilizer was dissolved in distilled water to prepare a 1 wt% polyvinyl alcohol mother liquid. Without adding a reducing agent, the ABTS mother liquid and the polyvinyl alcohol mother liquid were dissolved in distilled water together with a previously prepared 400 mM acetate buffer solution (pH = 4.5) to prepare 100 ml of enzyme solution (B8) having 8.0 mg/ml of ABTS, 0.05% of polyvinyl alcohol and 100 mM of acetate buffer solution. The enzyme solution (A8) and the substrate solution (B8) were each subjected to nitrogen bubbling under an airtight circumstance in a container with a check valve so that the concentration of dissolved oxygen was 0.00 mg/L. Then each of the solutions was fed to a mixer in an equal amount by a micro pump to continuously prepare an oxygen sensitive solution (C8) with the enzyme solution (A8) and the substrate solution (B8) mixed together. A filter paper bonded to the adhesive surface of an oxygen gas barrier adhesive label (PET manufactured by Sato Seal Co., Ltd., thickness: 75 µm) having an adhesive layer on one side as shown in FIG. 5 was impregnated with part of the oxygen sensitive solution (C8) under a low oxygen environment with the oxygen concentration of 30 ppm. The filter paper was covered with an oxygen permeable film (OPS film manufactured by Asahi Kasei Corporation, thickness: 25 µm) from above; bonded together with the adhesive force of the oxygen gas barrier adhesive label; covered with an oxygen gas barrier tape from above the oxygen permeable film; and bonded together with the adhesive force of the oxygen gas barrier adhesive label to fabricate an oxygen indicator (D8). Further, the obtained oxygen indicator (D8) was packaged with a bag made of an oxygen gas barrier film together with an oxygen absorbent. Then, in the same manner as in Example 2 except that the concentration of oxygen gas component in the measurement environment was adjusted to 0.0 vol%, 0.2 vol% and 0.5 vol% (measured with Check Point manufactured by DANCENSER Co., Ltd.), the obtained oxygen indicator (D8) was broken at the outer bag made of the oxygen gas barrier film in the measurement environment. The oxygen gas barrier tape was removed to detect the presence or absence of oxygen. The oxygen indicator was transparent in an oxygen concentration of 0.0% in the measurement environment and turned bluish green in an oxygen concentration of 0.2%, thus sharply indicating the presence of oxygen with a threshold of the oxygen concentration of 0.2%. The oxygen indicator (D8) was packaged with a bag made of the oxygen gas barrier film together with the oxygen absorbent and then stored at 5°C for 30 days after fabrication. The presence or absence of oxygen was detected in the same manner as described above using the stored oxygen indicator. The oxygen indicator was transparent in an oxygen concentration of 0.0% in the measurement environment and turned bluish green in an oxygen concentration of 0.2%, thus sharply indicating the presence of oxygen with a threshold of the oxygen concentration of 0.2%. There was no difference in oxygen detection capability of the oxygen indicator (D8) immediately after the fabrication and after the storage at 5°C for 30 days. It was found that the oxygen indicator was excellent in storage stability.

### INDUSTRIAL APPLICABILITY

The oxygen indicator of the present invention can be suitably used in applications of gas flush packaging where the presence of oxygen should be avoided.

## Claims

1. An oxygen indicator using an optical absorption spectral change reaction caused by a substrate in presence of oxygen via an enzymatic catalysis, which comprises an oxygen sensitive solution containing at least a coloring substrate at the substrate, an oxidoreductase, and a reducing agent capable of reducing the oxidized coloring substrate.

2. An oxygen indicator using an optical absorption spectral change reaction caused by a substrate in presence of oxygen via an enzymatic catalysis, which comprises an oxygen sensitive solution containing at least a coloring substrate at the substrate, an oxidoreductase and an enzyme stabilizer.

3. An oxygen indicator using an optical absorption spectral change reaction caused by a substrate in presence of oxygen via an enzymatic catalysis, which comprises an oxygen sensitive solution containing at least a coloring substrate at the substrate, an oxidoreductase, an enzyme stabilizer, and a reducing agent capable of reducing the oxidized coloring substrate.

4. The oxygen indicator according to claim 1 or 3, wherein said reducing agent is a mercapto group containing compound capable of producing a disulfide group when it is oxidized.

5. The oxygen indicator according to claim 2 or 3, wherein said enzyme stabilizer is a nonionic compound with a surface tension in a 0.2 wt% aqueous solution thereof equal to or less than 0.06 N/m.

6. The oxygen indicator according to claim 5, wherein said nonionic compound is a water-soluble polymer.

7. The oxygen indicator according to claim 6, wherein said water-soluble polymer is a water-soluble polyvinyl alcohol, water-soluble polyglycerin or water-soluble cellulose derivative.

8. The oxygen indicator according to any one of claims 5 to 7, wherein the oxidoreductase is ascorbate oxidase or bilirubin oxidase.

9. The oxygen indicator according to any one of claims 1 to 8, wherein said oxygen sensitive solution contains a buffer agent.

10. The oxygen indicator according to any one of claims 1 to 9, wherein said oxygen sensitive solution further contains a compound capable of reacting with oxygen in competition with said optical absorption spectral change reaction, or a compound capable of adsorbing oxygen.

11. A package comprising a container or bag, wherein the container or bag contains the oxygen indicator according to any one of claims 1 to 10, or the oxygen indicator according to any one of claims 1 to 10 is mounted in such a manner as to block the opening of the container or bag, whereby the concentration of oxygen in the container or bag can be detected.

12. The package according to claim 11, wherein the package has a form of vacuum packaging.

13. The package according to claim 11, wherein the package has a form of gas flush packaging with said container or bag filled with a gas containing no oxygen.
